(19) Europäisches Patentamt
European Patent Office
Office européen des brevets

(11) Numéro de publication: 0 060 734
B1

(12) # FASCICULE DE BREVET EUROPEEN

(45) Date de publication du fascicule du brevet: **06.03.85**

(51) Int. Cl.⁴: **C 07 C 69/533**, C 07 C 69/593, C 07 C 67/38

(21) Numéro de dépôt: **82400023.6**

(22) Date de dépôt: **08.01.82**

(54) **Procédé de préparation d'esters d'acides carboxyliques bêta-gamma insaturés.**

(30) Priorité: **23.01.81 FR 8101205**

(43) Date de publication de la demande: **22.09.82 Bulletin 82/38**

(45) Mention de la délivrance du brevet: **06.03.85 Bulletin 85/10**

(84) Etats contractants désignés: **AT BE CH DE FR GB IT LI LU NL SE**

(56) Documents cités:
**FR - A - 1 402 383**
**FR - A - 1 461 826**
**FR - A - 2 147 185**
**FR - A - 2 291 962**
**FR - A - 2 416 881**
**GB - A - 1 110 405**
**US - A - 3 657 368**

**BULLETIN OF THE CHEMICAL SOCIETY OF JAPAN, vol. 50, no. 2, 1977 J. TSUJI et al.: "Carbonylation reaction of isoprene catalyzed by palladium (II) actetate and triphenylphosphine", pages 553-554**
**BULLETIN DE LA SOCIETE CHIMIQUE DE FRANCE, vol. 9, 1968 J. SEYDEN-PENNE: "Notions d'acides et de bases durs et mous", pages 3871-3872**

(73) Titulaire: **RHONE-POULENC CHIMIE DE BASE, 25, quai Paul Doumer, F-92408 Courbevoie (FR)**

(72) Inventeur: **Jenck, Jean, 2, rue Lakanal, F-69100-Villeurbanne (FR)**

(74) Mandataire: **Varnière-Grange, Monique et al, RHONE-POULENC RECHERCHES Service Brevets Chimie et Polymères Centre de Recherches de Saint-Fons 85, rue des Frères Perret B.P. 62, F-69192 St-Fons Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

**Description**

La présente invention a pour objet un procédé de préparation d'esters d'acides carboxyliques $\beta,\gamma$-insaturés, par carbonylation de diènes conjugués par de l'oxyde de carbone en présence d'alcool.

Il est connu d'après le brevet japonais n° 485 564 de préparer des esters d'acides carboxyliques $\beta,\gamma$-insaturés, par carbonylation des diènes conjugués par de l'oxyde de carbone, en présence d'un monoalcool, d'un catalyseur au palladium non halogéné et d'un hydracide halogéné, à une température de l'ordre de 100°C et une pression d'oxyde de carbone de l'ordre de 100 bars.

A moins d'utiliser beaucoup de catalyseur et une forte concentration d'hydracide halogéné, ce procédé ne permet pas d'avoir en même temps une sélectivité élevée en esters recherchés, un taux élevé de conversion des diènes conjugués et une bonne stabilité du catalyseur au palladium.

Il a également été proposé (brevet français 1 461 826) de préparer des acides carboxyliques $\beta,\gamma$-insaturés ou leurs esters par carbonylation des diènes conjugués par de l'oxyde de carbone en milieu acide, en présence d'eau ou d'un alcool, d'un catalyseur au palladium déposé sur support et d'une phosphine organique. Ce procédé présente l'inconvénient de nécessiter des pressions élevées d'oxyde de carbone, par exemple de l'ordre de 700 bars.

Le brevet américain n° 4 172 087 décrit la synthèse d'esters de l'acide pentène-3 oïque en mélange avec une quantité importante d'esters de l'acide nonadiène-3,8 oïque, par carbonylation du butadiène par de l'oxyde de carbone en présence d'un alcool, d'une amine tertiaire hétérocyclique, d'un halogénure de palladium complexé par une phosphine tertiaire monodentée ou d'un sel de palladium non halogéné complexé par une phosphine tertiaire polydentée.

Un tel procédé est très peu sélectif en esters de l'acide pentène-3 oïque.

La demanderesse a trouvé un nouveau procédé de préparation des esters des acides carboxyliques $\beta,\gamma$-insaturés par carbonylation des diènes conjugués, permettant d'avoir une sélectivité élevée en esters recherchés, un taux de conversion amélioré des diènes conjugués mis en oeuvre, ainsi qu'une bonne stabilité du catalyseur au palladium, les températures et pressions d'oxyde de carbone mises en oeuvre étant du même ordre ou inférieures à celles normalement utilisées dans les opérations de carbonylation des diènes conjugués en présence de catalyseurs au palladium.

L'objet de l'invention est un procédé de préparation d'esters d'acides carboxyliques $\beta,\gamma$-insaturés, par carbonylation d'un diène conjugué par de l'oxyde de carbone, en présence de l'alcool correspondant à l'ester recherché, d'un hydracide halogéné et d'un catalyseur au palladium, à une température comprise entre 50 et 150°C et sous une pression d'oxyde de carbone de 50 à 300 bars, ledit procédé étant caractérisé en ce que:

- l'opération de carbonylation est réalisée en outre en présence d'un sel d'onium quaternaire d'un élément du groupe V B choisi parmi l'azote, le phosphore et l'arsenic, ledit élément étant quadricoordiné à des atomes de carbone et ledit sel présentant un anion choisi parmi les bases »dures« ou »intermédiaires«;
- le catalyseur au palladium est constitué par:
  - du palladium métal
  - un oxyde de palladium
  - ou un sel ou complexe de palladium dont l'anion coordiné au cation palladium est une base »dure« ou »intermédiaire«;
- le rapport molaire hydracide halogéné/palladium est d'au moins 5;
- le rapport molaire cation onium/palladium est d'au moins 0,5.

On entend par base »dure« ou »intermédiaire« tout anion répondant à la définition classique donnée par R. Pearson dans J. Chem. Ed, 45, 581-7 (1968).

Par cation onium quaternaire dont l'élément du groupe VB est quadricoordiné à des atomes de carbone, on entend des cations formés à partir d'azote, de phosphore ou d'arsenic et de quatre groupements hydrocarbonés monovalents, identiques ou différents, dont la valence libre est portée par un atome de carbone, chaque groupement étant relié à l'élément précité par ladite valence libre, deux quelconques de ces groupements pouvant par ailleurs former ensemble un radical unique divalent.

Pour une bonne réalisation du procédé de l'invention, le sel d'onium quaternaire présente un cation onium quaternaire répondant à l'une des formules I à III suivantes:

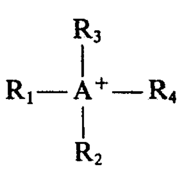

(I)

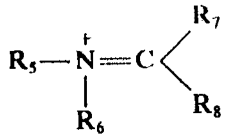

(II)

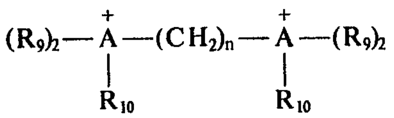

(III)

où:

– A représente de l'azote, du phosphore ou de l'arsenic
– $R_1$, $R_2$, $R_3$, $R_4$ sont identiques ou différents et représentent:

– un radical alkyle linéaire ou ramifié contenant de 1 à 16 atomes de carbone, éventuellement substitué par un groupe phényle, hydroxy, halogéno, nitro, alcoxy ou alcoxycarbonyle;
– un radical alcényle linéaire ou ramifié contenant de 2 à 12 atomes de carbone, de préférence de 4 à 8 atomes de carbone, et tout particulièrement un radical alcényle dérivé du diène conjugué mis en oeuvre;
– un radical aryle contenant de 6 à 10 atomes de carbone, éventuellement substitué par un ou des radicaux alkyles contenant de 1 à 4 atomes de carbone, alcoxy, alcoxycarbonyle ou halogéno;
– deux desdits radicaux $R_1$ à $R_4$ pouvant former ensemble un radical alkylène, alcénylène ou alcadiènylène, linéaire ou ramifié contenant de 3 à 6 atomes de carbone;

– $R_5$, $R_6$, $R_7$, $R_8$ sont identiques ou différents et représentent:

– un radical alkyle linéaire ou ramifié contenant de 1 à 4 atomes de carbone;
– les radicaux $R_7$ et $R_8$ pouvant former ensemble un radical alkylène contenant de 3 à 6 atomes de carbone;
– les radicaux $R_6$ et $R_7$ ou $R_6$ et $R_8$ pouvant former ensemble un radical alkylène, alcénylène ou alcadiénylène, contenant 4 atomes de carbone et constituant avec N un hétérocycle azoté;

– $R_9$ représente un radical alkyle linéaire ou ramifié contenant de 1 à 4 atomes de carbone ou un radical phényle;
– $R_{10}$ représente:

– un radical alkyle linéaire ou ramifié contenant de 1 à 4 atomes de carbone, semblable ou différent de $R_9$;
– un radicale alcényle linéaire ou ramifié, contenant de 2 à 12 atomes de carbone, de préférence de 4 à 8 atomes de carbone, et tout particulièrement un radical alcényle dérivé du diène conjugué à carbonyler;

– n représente un nombre entier supérieur ou égal à 1 et inférieur ou égal à 10 et de préférence inférieur ou égal à 6.

Parmi les bases »dures« ou »intermédiaires« pouvant constituer l'anion desdits sels d'onium, on peut citer les ions:

$F^-$, $ClO_4^-$, $PF_6^-$, $BF_4^-$, $B\Phi_4^-$, $PO_4^{3-}$, $HPO_4^{2-}$, $H_2PO_4^-$, $CH_3SO_3^-$, 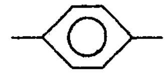$-SO_3^-$,

$HSO_4^-$, $NO_3^-$, $SO_4^{2-}$, $Cl^-$, $Br^-$,

ainsi que tous les autres anions répondant à la définition de base »dure« ou »intermédiaire« de Pearson.

Pour des raisons de commodité de mise en oeuvre, lesdits anions pourront être choisis parmi:

– $PO_4^{3-}$, $HPO_4^{2-}$, $H_2PO_4^-$, $CH_3SO_3^-$, 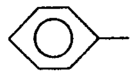$-SO_3^-$, $NO_3^-$, $SO_4^{2-}$, $PF_6^-$, $Cl^-$, $Br^-$

– de préférence $Cl^-$ et $Br^-$
– et en particulier $Cl^-$.

A titre d'exemple de cations onium quarternaire répondant à la formule I on peut citer les cations:

tétraméthylammonium,
triéthylméthylammonium,
tributylméthylammonium,
triméthyl(n-propyl)ammonium,
tétraéthylammonium,
tétrabutylammonium,
dodécyltriméthylammonium,
méthyltrioctylammonium,
heptyltributylammonium,
tétrapropylammonium,
tétrapentylammonium,
tétrahexylammonium,
tétraheptylammonium,
tétraoctylammonium,
tétradécylammonium,
butyltripropylammonium,
méthyltributylammonium,
pentyltributylammonium,
méthyldiéthylpropylammonium,
éthyldiméthylpropylammonium,
tétradodécylammonium,
tétraoctadécylammonium,
hexadécyltriméthylammonium,
benzyltriméthylammonium,
benzyldiméthylpropylammonium,
benzyldiméthyloctylammonium,
benzyltributylammonium,
benzyltriéthylammonium,
phényltriméthylammonium,
benzyldiméthyltétradécylammonium,
benzyldiméthylhexadécylammonium,
diméthyldiphénylammonium,
méthyltriphénylammonium,
butène-2 yltriéthylammonium,
N,N-diméthyl-tétraméthylènammonium,
N,N-diéthyl-tétraméthylènammonium,
tétraméthylphosphonium,
tétrabutylphosphonium,
éthyltriméthylphosphonium,
triméthylpentylphosphonium,
octyltriméthylphosphonium,
dodécyltriméthylphosphonium,
triméthylphénylphosphonium,
diéthyldiméthylphosphonium,
dicyclohexyldiméthylphosphonium,
diméthyldiphénylphosphonium,
cyclohexyltriméthylphosphonium,
triéthylméthylphosphonium,
méthyl-tri(isopropyl)phosphonium,
méthyl-tri(n-propyl)phosphonium,
méthyl-tri(n-butyl)phosphonium,
méthyl-tri(méthyl-2 propyl)phosphonium,
méthyltricyclohexylphosphonium,
méthyltriphénylphosphonium,
méthyltribenzylphosphonium,
méthyl-tri(méthyl-4 phényl)phosphonium,
méthyltrixylylphosphonium,
diéthylméthylphénylphosphonium,
dibenzylméthylphénylphosphonium,
éthyltriphénylphosphonium,
tétraéthylphosphonium,
éthyl-tri(n-propyl)phosphonium,
triéthylpentylphosphonium,

hexadécyltributylphosphonium,
éthyltriphénylphosphonium,
n-butyl-tri(n-propyl)phosphonium,
butyltriphénylphosphonium,
benzyltriphénylphosphonium,
(β-phényléthyl)diméthylphénylphosphonium,
tétraphénylphosphonium,
triphényl(méthyl-4 phényl)phosphonium,
tétrakis(hydroxyméthyl)phosphonium,
tétrakis(hydroxy-2 éthyl)phosphonium,
tétraphénylarsonium.

Parmi les cations répondant à la formule II, on peut citer les cations:

N-méthylpyridinium
N-éthylpyridinium
N-hexadécylpyridinium
N-méthylpicolinium.

Parmi les cations répondant à la formule III, on peut citer les cations:

bis(butène-2 yldiméthylammonium)-1,3 propane,
bis(triméthylammonium)-1,2 éthane,
bis(triméthylammonium)-1,3 propane,
bis(triméthylammonium)-1,4 butane,
bis(triméthylammonium)-1,3 butane.

Il peut être intéressant lorsque le sel d'onium choisi contient comme anion $Cl^-$ ou $Br^-$, et comme cation un cation de formule I ou III dans laquelle un des radicaux $R_1$ à $R_4$ ou le radical $R_{10}$ est un radical alcényle dérivant du diène conjugué mis en oeuvre, de préparer le sel d'onium »in situ« au lieu de le préparer préalablement à l'opération de carbonylation.

En effet il est particulièrement aisé de préparer ce type de produit par action d'une amine tertiaire par exemple sur le ou les produits de la réaction du diène conjugué mis en oeuvre sur l'acide chlorhydrique ou bromhydrique.

Ainsi lorsque le diène conjugué à carbonyler est du butadiène-1,3 on pourra utiliser comme sel d'onium quaternaire du chlorure ou du bromure de buténylalkylammonium, qu'il sera possible d'obtenir »in situ« dans le milieu de carbonylation, par action d'une alkylamine tertiaire sur du chloro-1 ou bromo-1 butène-2 ou sur du chloro-3 ou bromo-3 butène-1; si l'amine tertiaire est la triéthylamine par exemple, on pourra ainsi préparer »in situ« un chlorure ou un bromure de buténytriéthylammonium.

Parmi les catalyseurs au palladium pouvant être mis en oeuvre pour réaliser le procédé faisant l'objet de l'invention, on peut citer:

- le palladium métallique déposé sur un support, tel que le charbon, alumine, silice ......
- les oxydes de palladium
- les sels ou les complexes π-allyliques de palladium, dont l'anion coordiné au cation Pd est choisi parmi les anions suivants : carboxylates tels que formiate, acétate, propionate, benzoate; $SO_4^{2-}$, $NO_3^-$, acétylacétonate, halogénures tels que $Cl^-$, et $Br^-$ et de préférence $Cl^-$;
- ou les complexes de palladium zéro comprenant des ligands organiques ne contenant pas d'éléments du groupe VB, complexes tels que le bis(dibenzalacétone) Pd ou le bis(cyclooctadiène-1,5) Pd.

Le procédé faisant l'objet de l'invention est tout particulièrement intéressant pour la préparation d'esters carboxyliques β,γ-insaturés dérivés des diènes conjugués présentant dans leur molécule le squelette butadiène-1,3 et de monoalcools aliphatiques linéaires contenant de 1 à 3 atomes de carbone, de préférence 1 à 2 atomes de carbone, et tout particulièrement de l'éthanol.

Parmi les diènes conjugués présentant dans leur molécule le squelette butadiène-1,3, on peut citer:

- les diènes aliphatiques linéaires ou ramifiés, contenant de 4 à 12 atomes de carbone, et de préférence de 4 à 8 atomes de carbone éventuellement substitués par des groupes inertes tels que: phényle, cyclohexyle, nitro, oxo et en particulier alcoxycarbonyle;
- les diènes cycliques contenant de 6 à 8 atomes de carbone.

Comme exemples concrets de diène conjugué, on peut citer le butadiène-1,3, l'isoprène, le pipérylène, l'hexadiène-1,3, l'hexadiène-2,4, le chloroprène, le cyclohexyl-1 butadiène-1,3, le phényl-1 butadiène-1,3, l'octadiène-2,4, le méthyl-3 pentadiène-1,3 le méthyl-2 pentadiène-2,4, le cyclohexadiène-1,3, le cyclooctadiène-1,3, éventuellement substitués par un groupe alcoxycarbonyle, tel que pentadiène-2,4 oate de méthyle.

L'opération de carbonylation est favorablement réalisée en présence d'acide chlorhydrique ou bromhydrique, et tout particulièrement en présence d'HCl.

L'acide chlorhydrique peut être introduit dans le milieu de carbonylation sous forme gazeuse ou sous forme d'un composé organique susceptible de libérer de l'acide chlorhydrique dans le milieu, par exemple sous forme de chloro-1 butène-2 ou de chloro-3 butène-1 dans le cas de la carbonylation du butadiène.

Ceci présente l'avantage non négligeable d'éviter ou de diminuer les réactions secondaires de dégradation de l'alcool mis en oeuvre par l'acide chlorhydrique.

Les quantités de réactifs à utiliser pour réaliser le procédé faisant l'objet de la présente invention peuvent varier entre des limites très larges; il est évident que lesdites quantités seront choisies de manière à ce que le procédé soit économiquement intéressant.

Ainsi, bien qu'il soit possible d'utiliser de 0,5 à 10 fois la quantité d'alcool stoechiométriquement nécessaire, il est préférable afin d'avoir une conversion maximum du diène conjugué tout en évitant de trop diluer le milieu par de l'alcool, de réaliser le procédé avec un rapport molaire alcool/diène conjugué compris entre environ 0,8 et 5.

De même la bonne activité des catalyseurs au palladium permet l'emploi desdits catalyseurs en quantité très faible (correspondant à un rapport molaire diène conjugué/palladium de l'ordre de 2500); l'emploi d'une quantité élevée de catalyseur (correspondant à un rapport molaire diène conjugué/palladium de l'ordre de 100) n'est pas nuisible; le but recherché étant de réaliser une opération de carbonylation suffisamment rapide et sélective, sans dépenser trop de catalyseur, un rapport diène conjugué/palladium compris entre environ 250 et 2000 et en particulier entre environ 250 et 1200 est généralement préférable; il a été constaté qu'un rapport molaire compris entre environ 500 et 700 est particulièrement intéressant pour obtenir une conversion élevée des diènes conjugués, en particulier du butadiène, dans une durée raisonnable, ce qui permet d'éviter les réactions de dégradation.

La quantité d'hydracide halogéné à utiliser correspond à un rapport molaire hydracide halogéné/palladium d'au moins 5. Toutefois, afin d'éviter tout risque de précipitation du palladium sous forme de granules métalliques (précipitation due à une concentration trop faible en hydracide du milieu) ou de dégradation de l'alcool en chlorures d'alkyle et oxydes de dialkyle (dégradation due à une concentration trop importante du milieu en hydracide), un rapport molaire hydracide halogéné/palladium compris entre 10 et 150, et de préférence entre 20 et 100, pourra être favorablement choisi; le choix dudit rapport à l'intérieur de ces limites doit également tenir compte de la concentration du milieu en palladium; en effet l'homme de l'art sait que plus la concentration du milieu en palladium est élevée, moins un rapport hydracide/palladium élevé est nécessaire et inversement.

Il a été constaté que l'effet bénéfique apporté par la présence dans le milieu de carbonylation d'un sel d'onium quaternaire correspondant à la définition donnée ci-dessus est sensible à partir d'un rapport molaire cation onium/palladium de 0,5; notamment un effet particulièrement intéressant a été constaté lorsque ledit rapport est compris entre 1 et 15, un rapport plus élevé, n'étant toutefois pas nocif pour la réaction de carbonylation.

Por une bonne réalisation du procédé de carbonylation il sera préférable pour le choix du rapport cation onium/palladium de tenir compte de la concentration en palladium du milieu et notamment du rapport molaire diène conjugué/palladium; ainsi plus le rapport diène conjugué/palladium est élevé, plus il y a intérêt à utiliser un rapport cation onium/palladium élevé.

Les entités élémentaires correspondant aux termes »moles« sont les suivantes:

| | |
|---|---|
| alcool | molécule-gramme |
| diène conjugué | molécule-gramme |
| hydracide halogéné | molécule-gramme |
| palladium | atome-gramme |
| cation onium quaternaire | ion-gramme |

L'opération de carbonylation pourra généralement être réalisée à une température comprise entre 50 et 150°C, de préférence entre 80 et 130°C, sous une pression d'oxyde de carbone comprise entre 50 et 250 bars.

La température et la pression sont choisies en fonction de l'activité et/ou de la sélectivité recherchée pour un catalyseur donné, de manière à obtenir les meilleurs résultats possibles. En effet, il est connu que si la température diminue, la vitesse de conversion du diène diminue mais la sélectivité en monoester recherché augmente. Il a également été constaté que si la pression d'oxyde de carbone augmente, la sélectivité en monoester recherché augmente.

Le procédé faisant l'objet de l'invention peut être réalisé en continu ou en discontinu; on a constaté qu'on pouvait obtenir un taux de conversion du butadiène-1,3 d'au moins 70% en opérant en discontinu pendant 2 à 4 heures. Ledit procédé est peu sensible à la présence d'hydrogène ou de gaz inertes tels que azote, argon ou gaz carbonique qui peuvent être présents à côté de l'oxyde de carbone.

Les exemples suivants sont donnés à titre indicatif et ne peuvent être considérés comme une limite du

domaine et de l'esprit de l'invention.

Dans ces exemples, les abréviations figurant dans les tableaux I à X ci-dessus ont le sens suivant:

- Ex: exemple
- BD: butadiène
- EtOH: ethanol
- Me: méthyl, Et: éthyl, Oct: octyl, Φ: phényl
- catalyseur: catalyseur au palladium
- [<(—PdCl]$_2$: bis [π-allyl palladium (II) chlorure]
- cocatalyseur: HCl ou produit de la réaction HCl + diène conjugué mis en oeuvre
- additif: sel d'onium
- Pco: pression d'oxyde de carbone

  - L'absence de signe ↗ signifie que l'opération de carbonylation est réalisée à pression constante
  - La présence du signe ↗ signifie que l'opération de carbonylation est réalisée à pression non constante, en partant d'une pression de 120 bars à froid. Ainsi, ↗157 par exemple signifie que la pression d'oxyde de carbone est de 120 bars à froid et croit jusqu'à 157 bars à la température de l'opération de carbonylation puis diminue au fur et à mesure de l'avancement de la réaction.

Exemple 1—23

Préparation de pentène-3 oate d'éthyle
Emploi de différents sels d'onium

Mode opératoire général

Dans un autoclave de 125 cm$\alpha$ en alliage nickel-molybdène de marque Hastelloy $B_2$ on introduit sous courant d'argon, selon les quantités figurant aux tableaux I et II:

- le catalyseur à l'état anhydre [chlorure de palladium II ou bis[π-allyl palladium (II) chlorure];
- de l'éthanol absolu dans lequel on a solubilisé du gaz chlorhydrique ou additionné du chloro-1 butène-2 ou du chloro-3 butène-1;
- un additif maintenu à l'état anhydre correspondant ou non aux sels d'onium quaternaires dont l'emploi est revendiqué par la Demanderesse.

L'autoclave est ensuite fermé; on y transverse du butadiène selon les quantités indiquées aux tableaux I et II.

De l'oxyde de carbone est ensuite introduit. Cette introduction est effectuée différemment selon que l'opération de carbonylation est réalisée à pression constante ou a pression non constante.

Carbonylation à pression constante de CO

L'autoclave agité par secousses est porté à 120°C avec alimentation à pression constante (145 bars par exemple) de CO gazeux technique contenant environ 0,8% en volume d'hydrogène. On laisse la réaction se dérouler pendant 2 heures à cette température.

Carbonylation à pression non constante de CO

On charge dans l'autoclave 120 bars de CO technique et on porte à 120°C. La pression monte (par exemple jusqu'à 157 bars) dans l'autoclave. On laisse la réaction se dérouler pendant 2 heures avec baisse lente de la pression intérieure (jusqu'à 138 bars par exemple).

Après les deux heures de carbonylation, à pression constante ou non, tel qu'indiqué dans les tableaux I et II, l'autoclave est refroidi à 15°C et on dégaze lentement. La masse réactionelle, de couleur jaune citron, est analysée par chromatographie en phase gazeuse.

On constante ainsi la formation de composés suivants:

| | Abréviation |
|---|---|
| pentène-3 oate d'éthyle | $P_3$ |
| méthyl-2 butène-3 oate d'éthyle | p' |
| nonadiène-3,8 oate d'éthyle | $C_9$ |
| diesters éthyliques à 6 carbone (en majorité du méthyl-2 glutarate de diéthyle) | $C_6$ |

| | Abréviation |
|---|---|
| dimères de butadiène (essentiellement du vinyl-4 cyclohexène) | $HC_8$ |
| pentanoate d'éthyle<br>méthyl-2 butanoate d'éthyle | PA |
| éthoxy-3 butène-1<br>éthoxy-1 butène-2 | $ROC_4$ |
| pentène-4 oate d'éthyle | $P_4$ |
| chloro-3 butène-1<br>chloro-1 butène-2 | $ClC_4$ |
| chlorure d'éthyle<br>éther diéthylique (réaction parasite de HCl sur l'éthanol) | Cl |

Ainsi, à l'exemple 12, en partant de:

75 mg (0,423 mmoles) de $PdCl_2$
18 g (391 mmoles) d'éthanol
770 mg (21,1 mmoles) d'HCl
236 mg (0,85 mmoles) de chlorure de tétrabutylammonium
15 g (278 mmoles) de butadiène

et deux heures de carbonylation à 120°C, sous une pression non constante de CO de 158 bars, on obtient 34,7 g de masse réactionnelle jaune citron, dont l'analyse chromatographique en phase gazeuse donne le résultat suivant:

| | | |
|---|---|---|
| $P_3$ | 16,30 g | (127,3 mmoles) |
| P' | 0,56 g | (4,4 mmoles) |
| $C_9$ | 0,21 g | (1,15 mmoles) |
| $C_6$ | 0,13 g | (0,6 mmoles) |
| $HC_8$ | 0,18 g | (1,65 mmoles) |
| PA | traces | |
| $ROC_4$ | 0,16 g | (1,6 mmoles) |
| $P_4$ | traces | |
| $ClC_4$ | non calculé | |
| EtOH non transformé | 11,68 g | (253,9 mmoles) |
| chlorure d'éthyle + éther diéthylique | 1,56 g | (24,2 mmoles) |
| butadiène + traces de butène | 2,99 g | |

Les abréviations figurant dans les tableaux I' et II' (ainsi que III' à IX') ont le sens suivant:

TT: taux de conversion global du butadiène (en mole %);
RR: taux de conversion partiel (en mole %) pour chaque produit obtenu par rapport au butadiène chargé, avec $TT = \Sigma RR$.

Ne sont pris en compte pour le calcul de TT que les RR des produits suivants: $P_3$, $P_4$, P', $C_9$, $C_6$, $HC_8$, PA et $ROC_4$; en effet, les chlorobutènes ($ClC_4$) sont équivalents à un mélange butadiène + HCl carbonylable en $P_3$.

RT: sélectivité (en moles %) pour chaque produit avec $RT = \frac{RR}{TT}$
RRCl: taux de conversion partiel (en mole %) en chlorure d'éthyle et éther diéthylique, par rapport à l'éthanol chargé.
A: activité du catalyseur, exprimée en nombre de moles de $P_3$ obtenues par mole de Pd et par heure.

Analyse des résultats figurant aux tableaux I' et II'

— Les exemples 10 à 23 ont été réalisés selon la présente invention, avec les sels d'onium quaternaires revendiqués;
les sels d'onium quaternaires formés »in situ« aux exemples 20 et 21 ont pour formule:

$$(CH_3-CH=CH-CH_2)_2\overset{+}{N}(C_2H_5)_3Cl^-$$

et

$$[(CH_3-CH=CH-CH_2)\overset{+}{N}(CH_3)_2(CH_2)_3(CH_3)_2\overset{+}{N}(CH_2-CH=CH-CH_3)]2Cl^-$$

- les exemples 1 et 22 ont été réalisés sans additif;
- les exemples 2 à 9 ont été réalisés en présence d'additifs dont la formule ne correspond pas à celle des sels d'onium quaternaires revendiqués (soit par la nature du cation ou soit par celle de l'anion).

On constate que:

- l'emploi de sels d'onium non quaternaires n'apporte aucune amélioration quant à la sélectivité en $P_3$, par rapport à une opération effectuée sans additif;
- l'emploi d'un sel d'onium quaternaire dont l'anion n'est pas une hase dure ou intermédiaire, est néfaste;
- l'emploi d'un sel d'onium quaternaire tel que revendiqué apporte une amélioration quant aux paramètres RT en $P_3$, TT et A, et que cette amélioration est d'autant plus sensible que les radicaux hydrocarbonés liés à l'élément du groupe VB est plus gros.

## Exemples 24—27

### Préparation de pentène-3 oate d'éthyle
### Variation du rapport molaire additif/Pd

En suivant le mode opératoire général ci-dessus décrit, on réalise différentes opérations de carbonylation pendant deux heures à 120°C à l'acide des quantités de réactifs indiquées au tableau III, en faisant varier le rapport molaire chlorure de tétrabutylammonium/Pd de 0 à 10.

On constate (tableau III') que dans les conditions, le meilleur résultat est obtenu lorsque ledit rapport est égal à 3, notamment en ce qui concerne l'activité A qui est multipliée par 2 par rapport à une opération sans additif.

## Exemples 28—36

### Préparation du pentène-3 oate d'éthyle
### Variation du rapport HCl/Pd

En suivant le mode opératoire général ci-dessus décrit, on réalise différentes opérations de carbonylation pendant deux heures à 120°C, à l'aide des quantités de réactifs indiquées dans le tableau IV, en faisant varier le rapport HCl/Pd de 0 à50.

On constate (tableau IV'):

- qu'à concentration nulle ou faible en HCl, (exemples 29 et 31), l'emploi de l'additif revendiqué n'apporte guère d'amélioration et n'empêche pas la précipitation de Pd° ( ⊽ Pd°);
- que dans les conditions des essais la présence de l'additif revendiqué permet d'obtenir une stabilité du palladium métal, pour un rapport molaire HCl/Pd = 10 au lieu de 20 en l'absence d'additif.

## Exemples 37—41

### Préparation du pentène-3 oate d'éthyle
### Variation du rapport butadiène/Pd

En suivant le mode opératoire général ci-dessus décrit, on réalise différentes opérations de carbonylation pendant 2 heures à 120°C, à l'aide des quantités de réactifs indiqués dans le tableau V, en faisant varier le rapport molaire butadiène/Pd.

On constate (tableau V') que dans ces conditions:

- avec une concentration basse en Pd (BD/Pd = 1200 environ), les meilleurs résultats sont obtenus pour un rapport $NBu_n$ + $Cl^-$/Pd de 6, on observe une nette amélioration de la sélectivité en $P_3$;
- avec une concentration moyenne en Pd (BD/Pd = 500—600 environ), les meilleurs résultats sont obtenus pour un rapport $NBu_n^+Cl^-$/Pd de 3, où l'activité A est multipliée par 2;
- avec une concentration élevée en Pd (BD'Pd = 300 environ), on observe une très forte diminution de la quantité de $C_9$ formée (pratiquement inexistante).

## Exemples 42—46

### Préparation de pentène-3 oate d'éthyle
### Variation du rapport éthanol/butadiène

En suivant le mode opératoire général ci-dessus décrit, on réalise différentes opérations de carbonylation pendant 2 heures à 120°C, à l'aide des quantités de réactifs indiquées au tableau VI en faisant varier le rapport molaire éthanol/butadiène.

On constate (tableau VI') que dans ces conditions:

— pour un rapport EtOH/BD voisin de 1, l'addition d'un sel d'onium quaternaire selon l'invention permet d'augmenter A;
— lorsque ledit rapport est compris entre 1,5 et 1,8, l'addition dudit sel d'onium permet d'augmenter outre A, la sélectivité en $P_3$;
— lorsque ledit rapport est voisin de 4, la sélectivité en $P_3$ augmente considérablement.

## Exemples 47—50

### Préparation de pentène-3 oate d'éthyle
### Variation de la pression de CO

En suivant le mode opératoire général ci-dessus décrit, on réalise différentes opérations de carbonylation pendant 2 heures à 120°C, à l'aide des quantités de réactifs indiquées au tableau VII et dans les conditions de pression indiquées au tableau VII.

On constate (tableau VII'), que dans les conditions indiquées:

— l'emploi d'additif selon un rapport $NBu_4^+Cl^-$/Pd de 2 permet de diminuer la pression d'oxyde de carbone de 155 bars à 120 bars environ, tout en conservant la même activité A.

## Exemples 51—52

### Préparation de pentène-3 oate d'éthyle
### Variation de la température

En suivant le mode opératoire général ci-dessus décrit, on réalise différentes opérations de carbonylation, à l'aide des quantités de réactifs indiquées au tableau VIII, à une pression de l'ordre de 135 à 155 bars et dans les conditions de température indiquées au tableau VIII.

On constate (tableau VIII'):

— qu'à 120°C, même en présence d'une quantité inférieure de catalyseur au palladium, l'addition de $NBu_4^+Cl^-$, selon un rapport $NBu_4^+Cl^-/Pd = 3$, permet de doubler l'activité, et d'améliorer la sélectivité en $P_3$;
— qu'à 100°C, l'addition d'un sel d'onium quaternaire selon l'invention permet d'augmenter l'activité. Economiquement, il y a donc intérêt à opérer à une température légèrement supérieure pour augmenter la sélectivité en $P_3$.

## Exemples 53—54

### Préparation de pentène-3 oate de méthyle

En suivant le mode opératoire général ci-dessus décrit, on réalise la carbonylation du butadiène en présence de méthanol au lieu d'éthanol, en mettant en oeuvre les quantités de réactifs indiquées au tableau IX et dans les conditions de températures et de pression indiquées au tableau IX.

On constate que (tableau IX'):

— sans la présence d'additif la réaction de carbonylation est très lente, l'activité A et la sélectivité en $P_3$ sont très faibles, et ce même si la proportion de catalyseur est très importante;
— en présence d'additif ($NMeOct_3^+Cl^-$), on obtient une activité A et une sélectivité en $P_3$ intéressantes.

## Exemples 55—56

### Carbonylation de l'isoprène et du pipérylène

En suivant le mode opératoire général décrit ci-dessus, on réalise la carbonylation de l'isoprène d'une part et du pipérylène d'autre part, en présence d'éthanol, selon les conditions indiquées au tableau X.

L'analyse chromatographique des solutions obtenues indique la formation notamment des produits suivants:

Carbonylation de l'isoprène

méthyl-4 pentène-3 oate d'éthyle 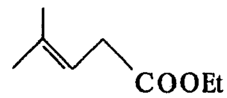

diméthyl-2,3 butène-2 oate d'éthyle 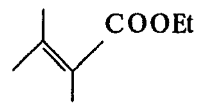

éthoxy-pentènes + diéthoxypentane

éthoxy-5 méthyl-4 pentanoate d'éthyle 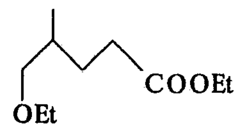

lactones

Carbonylation du pipérylène

méthyl-2 pentène-3 oate d'éthyle 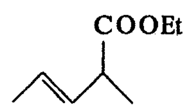

éthoxypentènes

dimères de pipérylène

éthoxydécènes

Les résultats des opérations de carbonylation sont indiqués dans le tableau X'.

## Exemple 57

### Carbonylation du pentadiène-2,4 oate de méthyle

En suivant le mode général opératoire ci-dessus décrit, on réalise une opération de carbonylation en mettant en oeuvre les quantités de réactifs suivantes:

| | | |
|---|---|---|
| pentadiène-2,4 oate de méthyle | 6,1 g | (54,4 mmoles) |
| méthanol | 3,48 g | (109 mmoles) |
| HCl | 350 mg | (9,6 mmoles) |
| $PdCl_2$ | 21,4 mg | (0,12 mmole) |
| $PBu_4Cl$ | 180 mg | (0,61 mmoles) |

L'opération est réalisée pendant 2 heures à 100°C sous une pression de 180 bars. On récupère 10,3 g d'une solution homogène de couleur jaune citron.

Les résultats de l'analyse chromatographique en phase gazeuse de la solution obtenue sont les suivants:

– 4 g de produits de carbonylation (diesters insaturés en $C_6$) constitués de:

41% de 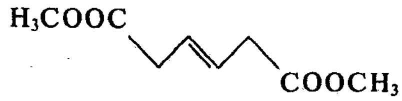

39% de 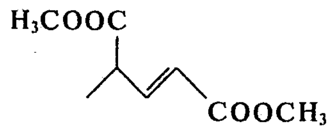

15% de 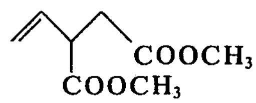

5% de 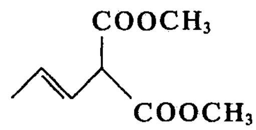

– et 0,9 g de dimères du pentadiène-2,4 oate de méthyle.

Le taux de conversion partiel RR en diesters insaturés est de 43 moles %, ce qui correspond à une activité spécifique, calculée sur tous les diesters insaturés de 97 $h^{-1}$.

## Exemple 58

### Préparation de pentène-3 oate d'éthyle
### Emploi d'un sel d'onium quaternaire non revendiqué: $NEt_4SnCl_3$

En suivant le mode opératoire décrit ci-dessus on réalise une opération de carbonylation en mettant en oeuvre les quantités de réactifs suivantes:

| | | |
|---|---|---|
| butadiène | 14,5 g | (268,5 mmoles) |
| bis ($\pi$-allyl palladium chlorure) | 0,077 g | (0,423 mmole) |
| éthanol | 18 g | (391,3 mmoles) |
| chlorure de crotyle | 1,91 g | (21,15 mmoles) |
| $NEt_4SnCl_3$ | 0,3013 g | (0,846 mmole) |

L'opération est réalisée pendant 2 heures à 120°C sous une pression d'oxyde de carbone non constante de 150 bars.

On récupère 21,8 g d'une solution jaune clair qui contient du palladium précipité.

Le résultat de l'analyse chromatographique en phase gazeuse de la solution obtenue est la suivante:

| Produits | Poids g | mmoles | RT% | RR% |
|---|---|---|---|---|
| Ethoxybutène | 0,33 | 3,3 | 12,1 | 1,2 |
| Vinylcyclohexène | 0,30 | 5,6 | 20,6 | 2 |
| méthyl-2 butène-3 oate d'éthyle | 0,07 | 0,5 | 1,8 | 0,2 |
| $P_3$ | 2,28 | 17,8 | 65,4 | 6,6 |

L'activité spécifique A est de 21 $h^{-1}$ et le taux de transformation TT du butadiène est de 10,1 moles %.

Exemple 59

Préparation du pentène-3 oate d'éthyle
Emploi d'un mélange $NEt_4SnCl_3$ et de triphénylphosphine non revendiqué

En suivant le mode opératoire décrit ci-dessus on réalise une opération de carbonylation en mettant en oeuvre les quantités de réactifs suivantes:

| | | |
|---|---|---|
| butadiène | 14 g | (259,2 mmoles) |
| bis ($\pi$ allyl palladium chlorure) | 0,0774 g | (0,423 mmole) |
| éthanol | 18 g | (391,3 mmoles) |
| HCl gazeux | 0,77 g | (21,1 mmoles) |
| $NEt_4SnCl_3$ | 0,3013 g | (0,846 mmole) |
| triphénylphosphine | 0,1109 g | (0,423 mmole) |

L'opération est réalisée pendant 2 heures à 120°C sous une pression d'oxyde de carbone non constante de 150 bars.

On récupère 20,5 g d'une solution jaune clair contenant du palladium précipité.

Le résultat de l'analyse chromatographique en phase gazeuse de la solution obtenue est la suivante:

| Produits | Poids g | mmoles | RT% | RR% |
|---|---|---|---|---|
| Ethoxy-butène | 0,28 | 2,8 | 13,7 | 1 |
| Vinylcyclohexène | 0,21 | 3,9 | 19 | 1,5 |
| méthyl-2 butène-3 oate d'éthyle | 0,05 | 0,4 | 2 | 0,1 |
| $P_3$ | 1,71 | 13,4 | 65,4 | 5,1 |

L'activité spécifique A est de 16 $h^{-1}$ et le taux de transformation TT du butadiène de 7,9 moles %.

Tableau I

| EX | BD mM | EtOH mM | Catalyseur mM $PdCl_2$ | Cocatalyseur mM HCl | BD/Pd molaire | HCl/Pd molaire | Additif | Additif/Pd molaire | PCO bars |
|---|---|---|---|---|---|---|---|---|---|
| 1 | 213 | 391 | 0,423 | 21,1 | 504 | 50 | – | 0 | ↗157 |
| 2 | 241 | 391 | 0,423 | 21,1 | 570 | 50 | $NH_4^+Cl^-$ | 2 | ↗153 |
| 3 | 250 | 391 | 0,423 | 21,1 | 592 | 50 | $NEt_3$ | 2 | ↗157 |
| 4 | 278 | 391 | 0,423 | 21,1 | 657 | 50 | $\Phi_3P = N^+ = P\Phi_3Cl^-$ | 5 | ↗148 |
| 5*) | 278 | 470 | 0,56 | 60 | 500 | 110 | $P\Phi_3$ | 2 | ↗158 |
| 6 | 287 | 391 | 0,423 | 21,1 | 679 | 50 | $NBu_4^+I^-$ | 10 | ↗157 |
| 7 | 269 | 391 | 0,423 | 21,1 | 636 | 50 | $NBu_4^+SCN^-$ | 2 | 145 |
| 8 | 259 | 391 | 0,423 | 21,1 | 613 | 50 | $MeP\Phi_3^+I^-$ | 10 | ↗154 |
| 9 | 278 | 391 | 0,423 | 42,3 | 657 | 100 | $MeP\Phi_3^+I^-$ | 3 | ↗153 |

Suite

| EX | BD mM | EtOH mM | Catalyseur mM $PdCl_2$ | Cocatalyseur mM HCl | BD/Pd molaire | HCl/Pd molaire | Additif | Additif/Pd molaire | PCO bars |
|---|---|---|---|---|---|---|---|---|---|
| 10 | 222 | 391 | 0,423 | 21,1 | 526 | 50 | $NMe_4^+Cl^-$ | 2 | ↗157 |
| 11 | 250 | 391 | 0,423 | 21,1 | 592 | 50 | $NEt_4^+Cl^-$ | 2 | ↗151 |
| 12 | 278 | 391 | 0,423 | 21,1 | 657 | 50 | $NBu_4^+Cl^-$ | 2 | ↗158 |
| 13 | 278 | 391 | 0,423 | 21,1 | 657 | 50 | $NMeOct_3^+Cl^-$ | 2 | ↗158 |
| 14 | 241 | 391 | 0,423 | 21,1 | 570 | 50 | $PBu_4^+Cl^-$ | 2 | ↗150 |
| 15 | 259 | 391 | 0,423 | 21,1 | 614 | 50 | $As\Phi_4^+Cl^-$ | 2 | ↗152 |

*) Réaction pendant 3 h 20 au lieu de 2 heures.

Tableau I'

| EX | Additif | TT % | A | P3 RT % | P3 RR % | P' RT % | P' RR % | C9 RT % | C9 RR % | HC8 RT % | HC8 RR % | ROC4 RT % | ROC4 RR % | C6 RT % | C6 RR % | RR Cl % |
|---|---|---|---|---|---|---|---|---|---|---|---|---|---|---|---|---|
| 1 | – | 37,4 | 84 | 89,6 | 33,5 | 2,6 | 1 | 2,5 | 0,9 | 3,5 | 1,3 | 1,8 | 0,7 | ε | ε | 5,5 |
| 2 | $NH_4^+Cl^-$ | 41,2 | 104 | 88,8 | 36,6 | 3 | 1,2 | 2,7 | 1,1 | 3,5 | 1,4 | 1,7 | 0,7 | 0,2 | 0,1 | 6,4 |
| 3 | $NEt_3$ | 40,2 | 99 | 87 | 34,8 | 3,6 | 1,6 | 3,6 | 1,6 | 2,9 | 1,2 | ε | ε | 2,4 | 1 | 2,9 |
| 4 | $\Phi_3P=\overset{+}{N}=P\Phi_3Cl^-$ | 29,8 | 82 | 87,3 | 25,3 | 2,9 | 0,8 | 5 | 1,4 | 4,1 | 1,2 | ε | ε | 0,6 | 0,2 | 7,4 |
| 5 | $P\Phi_3$ | 11,8 | 5 | faible | | | | | | | | | | | | |
| 6 | $NBu_4^+I^-$ | 5,5 | 12 | 63,3 | 3,5 | ε | ε | ε | ε | 31,6 | 1,7 | 5 | 0,3 | ε | ε | 5,1 |
| 7 | $NBu_4^+SCN^-$ | 10 | 22 | 69 | 6,9 | 2,2 | 0,2 | ε | ε | 23,2 | 2,3 | 5,9 | 0,6 | ε | ε | 1 |
| 8 | $MeP\Phi_3^+I^-$ | 5,8 | 6 | 43,9 | 2,5 | ε | ε | 2,7 | 0,1 | 42,6 | 2,4 | 10,8 | 0,6 | 0,8 | 0,1 | 3,3 |
| 9 | $MeP\Phi_3^+I^-$ | 9,4 | 20 | 67,9 | 6,4 | ε | ε | ε | ε | 24 | 2,2 | 8 | 0,7 | ε | ε | 5,3 |
| 10 | $NMe_4^+Cl^-$ | 57,6 | 137 | 90,5 | 52,2 | 3,2 | 1,8 | 3,3 | 1,9 | 2,2 | 1,3 | ε | ε | 0,8 | 0,5 | 6 |
| 11 | $NEt_4^+Cl^-$ | 53,6 | 145 | 91,3 | 48,8 | 3 | 1,6 | 2,8 | 1,5 | 2,5 | 1,3 | ε | ε | 0,6 | 0,3 | 5,8 |
| 12 | $NBu_4^+Cl^-$ | 50,4 | 151 | 91,3 | 46 | 3,1 | 1,6 | 1,6 | 0,8 | 2,3 | 1,2 | 1,1 | 0,6 | 0,4 | 0,2 | 6,2 |
| 13 | $NMeOct_3^+Cl^-$ | 55,9 | 168 | 91,9 | 51,4 | 3,1 | 1,7 | 1,9 | 1,1 | 2,7 | 1,5 | ε | ε | 0,4 | 0,3 | 7,1 |
| 14 | $PBu_4^+Cl^-$ | 57,3 | 152 | 93,3 | 53,5 | 2,5 | 1,5 | 2,2 | 1,3 | 1 | 0,6 | ε | ε | 0,8 | 0,5 | 6 |
| 15 | $As\Phi_4^+Cl^-$ | 43,2 | 123 | 92,6 | 40 | 2,9 | 1,3 | 1,2 | 0,2 | 1,7 | 0,7 | 1,6 | 0,7 | ε | ε | 6 |

Tableau II

| EX | BD mM | EtOH mM | Catalyseur mM $PdCl_2$ | Cocatalyseur mM HCl | BD/Pd molaire | HCl/Pd molaire | Additif | Additif/Pd molaire | PCO bars |
|---|---|---|---|---|---|---|---|---|---|
| 16 | 287 | 391 | 0,423 | 21,1 | 679 | 50 | $NBu_4^+Br^-$ | 2 | ↗157 |
| 17 | 278 | 391 | 0,423 | 21,1 | 657 | 50 | $NBu_4^+PF_6^-$ | 2 | ↗156 |
| 18 | 250 | 391 | 0,423 | 21,1 | 592 | 50 | $NBu_4^+CH_3SO_3^-$ | 2 | 145 |
| 19 | 259 | 391 | 0,423 | 21,1 | 612 | 50 | $NBu_4^+H_2PO^-$ | 2 | 145 |
| | | | | 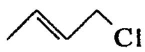 | | | | | |
| 20 | 287 | 391 | 0,423 | 21,1 | 679 | | $NEt_3$ | 2 | ↗157 |
| 21 | 260 | 391 | 0,423 | 21,1 | 613 | 50 | $Me_2N-(CH_2)_3-NMe_2$ | 2 | ↗157 |
| | | | [<(—PdCl]$_2$ | | | | | | |
| 22 | 250 | 391 | 0,423/2 | 21,1 | 591 | 50 | – | – | 145 |
| 23 | 259 | 391 | 0,423/2 | 21,1 | 613 | 50 | $PBu_4^+Cl^-$ | 2 | 145 |

Tableau II'

| EX | Additif | TT % | A | P3 RT % | P3 RR % | P' RT % | P' RR % | C9 RT % | C9 RR % | HC8 RT % | HC8 RR % | ROC4 RT % | ROC4 RR % | C6 RT % | C6 RR % | RR Cl % |
|---|---|---|---|---|---|---|---|---|---|---|---|---|---|---|---|---|
| 16 | $NBu_4^+Br^-$ | 48,7 | 147 | 89,9 | 43,7 | 3,2 | 1,6 | 3 | 1,5 | 3,7 | 1,8 | ε | ε | ε | ε | 5,2 |
| 17 | $NBu_4^+PF_6^-$ | 51 | 150 | 91,2 | 45,5 | 3,2 | 1,6 | 2,6 | 1,3 | 2,7 | 1,3 | ε | ε | 0,4 | 0,2 | 7 |
| 18 | $NBu_4^+CH_3SO_3^-$ | 56,7 | 152 | 90,5 | 51,3 | 3,1 | 1,8 | 2,3 | 1,3 | 2,9 | 1,7 | 0,3 | 0,2 | 0,8 | 0,4 | 2,4 |
| 19 | $NBu_4^+H_2PO_4^-$ | 61,4 | 171 | 90,9 | 55,8 | 3,4 | 2,1 | 2,4 | 1,5 | 1,9 | 1,2 | 0,1 | ε | 1,1 | 0,7 | 2,6 |
| 20 | $NEt_3^+$ 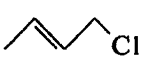 | 50,6 | 156 | 90,9 | 46 | 3,1 | 1,6 | 2,1 | 1,1 | 2,8 | 1,4 | 0,3 | 0,2 | 0,7 | 0,4 | 2,1 |
| 21 | $Me_2N-(CH_2)_3-NM_2$ + 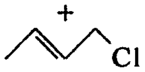 | 52 | 143 | 89,4 | 46,5 | 3 | 1,5 | 3,2 | 1,7 | 1,9 | 1 | 0,3 | 0,2 | 2 | 1 | 2,1 |
| 22 | – | 40,6 | 107 | 90,5 | 36,8 | 2,9 | 1,2 | 2,1 | 0,9 | 3,8 | 1,6 | 0,1 | ε | 0,4 | 0,2 | 2,3 |
| 23 | $PBu_4^+Cl^-$ | 58,3 | 164 | 91,7 | 53,4 | 3,2 | 1,9 | 2 | 1,1 | 2 | 1,1 | 0,2 | 0,1 | 0,9 | 0,5 | 1,8 |

Tableau III

| EX | BD mM | EtOH mM | $PdCl_2$ mM | HCl mM | BD/PD molaire | HCl/Pd molaire | $NBu_4^+Cl^-$/Pd molaire | PCO bars |
|---|---|---|---|---|---|---|---|---|
| 1 | 213 | 391 | 0,423 | 21,1 | 504 | 50 | 0 | ↗157 |
| 24 | 222 | 391 | 0,423 | 21,1 | 526 | 50 | 1 | ↗154 |
| 12 | 278 | 391 | 0,423 | 21,1 | 657 | 50 | 2 | ↗158 |
| 25 | 259 | 391 | 0,423 | 21,1 | 613 | 50 | 3 | 145 |
| 26 | 250 | 391 | 0,423 | 21,1 | 592 | 50 | 5 | ↗154 |
| 27 | 259 | 391 | 0,423 | 21,1 | 613 | 50 | 10 | ↗157 |

Tableau III'

| EX | TT % | A | P3 | | P' | | C9 | | HC8 | | ROC4 | | C6 | | RRCl % |
|---|---|---|---|---|---|---|---|---|---|---|---|---|---|---|---|
| | | | RT % | RR % | RT % | RR % | RT % | RR % | RT % | RR % | RT % | RR % | RT % | RR % | |
| 1 | 37,4 | 84 | 89,6 | 33,5 | 2,6 | 1 | 2,5 | 0,9 | 3,5 | 1,3 | 1,8 | 0,7 | ε | ε | 5,5 |
| 24 | 50,7 | 121 | 91 | 46,1 | 3,3 | 1,7 | 3,1 | 1,6 | 2,1 | 1 | ε | ε | 0,5 | 0,3 | 6,2 |
| 12 | 50,4 | 151 | 91,3 | 46 | 3,1 | 1,6 | 1,6 | 0,8 | 2,3 | 1,2 | 1,1 | 0,6 | 0,4 | 0,2 | 6,2 |
| 25 | 55,3 | 156 | 91,8 | 50,9 | 2,5 | 1,4 | 1,8 | 1 | 1,3 | 0,7 | ε | ε | 1,5 | 0,8 | 3,7 |
| 26 | 48 | 128 | 90,8 | 43,7 | 2,8 | 1,4 | 3,8 | 1,8 | 2,6 | 1,2 | ε | ε | ε | ε | 6 |
| 27 | 49,6 | 129 | 86,5 | 42,8 | 2,6 | 1,3 | 6,8 | 3,4 | 3,3 | 1,6 | ε | ε | ε | ε | 8 |

Tableau IV

| EX | BD mM | EtOH mM | Catalyseur mM | Cocatalyseur mM HCl | BD/Pd molaire | HCl/Pd molaire | Additif | Additif/Pd molaire | PCO bars |
|---|---|---|---|---|---|---|---|---|---|
| | | | [<(—PdCl]$_2$ | | | | $NBu_4^+Cl^-$ | | |
| 28 | 241 | 391 | 0,423/2 | 0 | 569 | 0 | 0 | 0 | ↗157 |
| 29 | 241 | 391 | 0,423/2 | 0 | 569 | 0 | 2,11 | 5 | ↗152 |
| 30 | 222 | 391 | 0,423/2 | 1,35 | 526 | 3,2 | 0 | 0 | 145 |
| 31 | 222 | 391 | 0,423/2 | 1,35 | 526 | 3,2 | 2,11 | 5 | ↗157 |

Suite

| EX | BD mM | EtOH mM | Catalyseur mM | Cocatalyseur mM | BD/Pd molaire | HCl/Pd molaire | Additif | Additif/Pd molaire | PCO bars |
|---|---|---|---|---|---|---|---|---|---|
| | | | | HCl | | | | | |
| | | | $PdCl_2$ | | | | | | |
| 32 | 222 | 391 | 0,423 | 4,23 | 526 | 10 | 0 | 0 | ↗153 |
| 33 | 241 | 391 | 0,423 | 4,23 | 570 | 10 | 1,27 | 3 | 145 |
| 34 | 232 | 391 | 0,423 | 4,23 | 549 | 10 | 8,45 | 20 | 145 |
| 35 | 222 | 391 | 0,423 | 8,45 | 526 | 20 | 0 | 0 | ↗152 |
| 36 | 222 | 391 | 0,423 | 8,45 | 526 | 20 | 1,27 | 3 | 145 |
| 1 | 213 | 391 | 0,423 | 21,1 | 504 | 50 | 0 | 0 | ↗157 |
| 25 | 259 | 391 | 0,423 | 21,1 | 613 | 50 | 1,27 | 3 | 145 |

Tableau IV'

| EX | HCl/Pd molaire | TT % | A | P3 | | P' | | C9 | | HC8 | | ROC4 | | C6 | | RR Cl % | ⌝↓ Pd° |
|---|---|---|---|---|---|---|---|---|---|---|---|---|---|---|---|---|---|
| | | | | RT % | RR % | RT % | RR % | RT % | RR % | RT % | RR % | RT % | RR % | RT % | RR % | | |
| 28 | 0 | 7,1 | 6,5 | 32,2 | 2,3 | 2,3 | 0,2 | 28,6 | 2 | 28 | 2 | 8,8 | 0,6 | ε | ε | ε | Oui |
| 29 | 0 | 5,5 | 5 | 31,1 | 1,7 | ε | ε | 30,4 | 1,7 | 38,5 | 2,1 | ε | ε | ε | ε | ε | Oui |
| 30 | 3,2 | 22,2 | 46 | 79,4 | 17,6 | 1,8 | 0,4 | 12 | 2,6 | 4,5 | 1 | 2,2 | 0,5 | ε | ε | 0,4 | Oui |
| 31 | 3,2 | 26,7 | 56 | 80,4 | 21,5 | 2,8 | 0,8 | 13,1 | 3,5 | 3,7 | 1 | ε | ε | ε | ε | ε | Oui |
| 32 | 10 | 19 | 35 | 70,2 | 13,4 | 1,4 | 0,2 | 13,1 | 2,5 | 10,8 | 2 | 4,5 | 0,8 | ε | ε | 0,3 | Oui |
| 33 | 10 | 45,9 | 110 | 83,5 | 38,3 | 2,4 | 0,7 | 10,3 | 2,9 | 1,8 | 0,5 | 0,2 | ε | 1,6 | 0,5 | 1,2 | Non |
| 34 | 10 | 47,9 | 108 | 82,3 | 39,4 | 1,8 | 0,9 | 11,9 | 5,7 | 2 | 1 | ε | ε | 1,4 | 0,7 | 1,1 | Non |
| 35 | 20 | 33,5 | 76 | 86,3 | 28,9 | 2,1 | 0,7 | 5,5 | 1,9 | 4,4 | 1,5 | 1,6 | 0,5 | ε | ε | 2,2 | Non |
| 36 | 20 | 53,9 | 130 | 92,6 | 49,5 | 0,3 | 0,1 | 4,1 | 2,2 | 1,4 | 0,8 | ε | ε | 0,9 | 0,5 | 1,9 | Non |
| 1 | 50 | 37,4 | 84 | 89,6 | 38,5 | 2,6 | 1 | 2,5 | 0,9 | 3,5 | 1,3 | 1,8 | 0,7 | ε | ε | 5,5 | Non |
| 25 | 50 | 55,3 | 156 | 91,8 | 50,9 | 2,5 | 1,4 | 1,8 | 1 | 1,3 | 0,7 | ε | ε | 1,5 | 0,8 | 3,7 | Non |

Tableau V

| EX | BD mM | EtOH mM | $PdCl_2$ mM | HCl mM | BD/Pd molaire | HCl/Pd molaire | $NBu_4^+Cl^-$ mM | $NBu_4^+Cl^-$/Pd molaire | PCO bars |
|---|---|---|---|---|---|---|---|---|---|
| 37 | 204 | 391 | 0,212 | 10,6 | 960 | 50 | 0 | 0 | →151 |
| 38 | 259 | 391 | 0,212 | 10,6 | 1229 | 50 | 0,423 | 2 | 145 |
| 39 | 250 | 391 | 0,212 | 10,6 | 1184 | 50 | 1,266 | 6 | 145 |
| 1 | 213 | 391 | 0,423 | 21,1 | 504 | 50 | 0 | 0 | →157 |
| 24 | 222 | 391 | 0,423 | 21,1 | 526 | 50 | 0,42 | 1 | →154 |
| 25 | 259 | 391 | 0,423 | 21,1 | 613 | 50 | 1,27 | 3 | 145 |
| 27 | 259 | 391 | 0,423 | 21,1 | 613 | 50 | 4,2 | 10 | →157 |
| 40 | 222 | 391 | 0,845 | 42,3 | 263 | 50 | 0 | 0 | →154 |
| 41 | 250 | 391 | 0,845 | 42,3 | 296 | 50 | 1,69 | 2 | 145 |

Tableau V'

| EX | Bd/Pd molaire | TT % | A | P3 RT % | P3 RR % | P' RT % | P' RR % | C9 RT % | C9 RR % | HC8 RT % | HC8 RR % | ROC4 RT % | ROC4 RR % | C6 RT % | C6 RR % | RR Cl % |
|---|---|---|---|---|---|---|---|---|---|---|---|---|---|---|---|---|
| 37 | 960 | 22 | 80 | 77,4 | 17 | 1,8 | 0,4 | 4,9 | 1 | 8,3 | 1,8 | 7,6 | 1,7 | ε | ε | 2,4 |
| 38 | 1129 | 22 | 116 | 86,3 | 19 | 2,3 | 0,5 | 3,9 | 0,8 | 5,8 | 1,3 | 1,8 | 0,4 | ε | ε | 1,8 |
| 39 | 1184 | 29 | 150 | 87,6 | 25,4 | 2,5 | 0,7 | 5,2 | 1,5 | 3,9 | 1,1 | 0,7 | 0,2 | ε | ε | 2,5 |
| 1 | 504 | 37,4 | 84 | 89,6 | 33,5 | 2,6 | 1 | 2,5 | 0,9 | 3,5 | 1,3 | 1,8 | 0,7 | ε | ε | 5,5 |
| 24 | 526 | 50,7 | 121 | 91 | 46,1 | 3,3 | 1,7 | 3,1 | 1,6 | 2,1 | 1 | ε | ε | 0,5 | 0,3 | 6,2 |
| 25 | 613 | 55,3 | 156 | 91,8 | 50,9 | 2,5 | 1,4 | 1,8 | 1 | 1,3 | 0,7 | ε | ε | 1,5 | 0,8 | 3,7 |
| 27 | 613 | 49,6 | 129 | 86,4 | 42,8 | 2,6 | 1,3 | 6,8 | 3,4 | 3,3 | 1,6 | ε | ε | ε | ε | 8 |
| 40 | 263 | 66,3 | 80 | 92 | 61,1 | 2,9 | 1,9 | 0,9 | 0,6 | 2,7 | 1,8 | ε | ε | 1,5 | 1 | 22 |
| 41 | 296 | 41,8 | 58 | 93,7 | 39,2 | 2,2 | 0,9 | ε | ε | 1 | 0,4 | ε | ε | 0,9 | 0,4 | 5,3 |

Tableau VI

| EX | BD mM | EtOH mM | Catalyseur mM | Cocatalyseur mM | BD/Pd molaire | EtOH/Bd molaire | HCl/Pd molaire | Additif mM | Additif/Pd molaire | PCO bars |
|---|---|---|---|---|---|---|---|---|---|---|
| | | | $PdCl_2$ | HCl | | | | $PBu_4^+Cl^-$ | | |
| 42 | 269 | 261 | 0,423 | 21,1 | 636 | 0,97 | 50 | 0 | 0 | 145 |
| 43 | 278 | 261 | 0,423 | 21,1 | 658 | 0,94 | 50 | 0,423 | 1 | 145 |
| | | | | | | | | $NBu_4^+Cl^-$ | | |
| 35 | 222 | 391 | 0,423 | 8,45 | 526 | 1,76 | 20 | 0 | 0 | →152 |
| 36 | 222 | 391 | 0,423 | 8,45 | 526 | 1,76 | 20 | 1,27 | 3 | 145 |
| 1 | 213 | 391 | 0,423 | 21,1 | 504 | 1,84 | 50 | 0 | 0 | →157 |
| 25 | 259 | 391 | 0,423 | 21,1 | 613 | 1,51 | 50 | 1,27 | 3 | 145 |
| 44 | 185 | 800 | 0,34 | 6,8 | 544 | 4,3 | 20 | 0 | 0 | 145 |
| 45 | 194 | 800 | 0,34 | 6,8 | 570 | 4,1 | 20 | 1,02 | 3 | 145 |
| 46 | 185 | 800 | 0,34 | 6,8 | 544 | 4,3 | 20 | 4,1 | 12 | 145 |

Tableau VI'

| EX | EtOH/Bd molaire | TT % | A | P3 | | P' | | C9 | | HC8 | | ROC4 | | C6 | | RR Cl % |
|---|---|---|---|---|---|---|---|---|---|---|---|---|---|---|---|---|
| | | | | RT % | RR % | RT % | RR % | RT % | RR % | RT % | RR % | RT % | RR % | RT % | RR % | |
| 42 | 0,97 | 43,8 | 127 | 91,1 | 39,9 | 2,2 | 1 | 1,7 | 0,7 | 3 | 1,3 | 0,8 | 0,3 | 1,3 | 0,6 | 3 |
| 43 | 0,94 | 46,3 | 139 | 91,1 | 42,2 | 2,5 | 1,2 | 3 | 1,5 | 2,6 | 1,2 | 0,9 | 0,4 | ε | ε | 2,7 |
| 35 | 1,76 | 33,9 | 76 | 86,3 | 28,9 | 2,1 | 0,7 | 5,5 | 1,9 | 4,4 | 1,5 | 1,6 | 0,5 | ε | ε | 2,2 |
| 36 | 1,76 | 53,9 | 130 | 92,6 | 49,5 | 0,3 | 0,1 | 4,1 | 2,2 | 1,4 | 0,8 | ε | ε | 0,9 | 0,5 | 1,9 |
| 1 | 1,84 | 37,4 | 84 | 89,6 | 33,5 | 2,6 | 1 | 2,5 | 0,9 | 3,5 | 1,3 | 1,8 | 0,7 | ε | ε | 5,5 |
| 25 | 1,51 | 55,3 | 156 | 91,8 | 50,9 | 2,5 | 1,4 | 1,8 | 1 | 1,3 | 0,7 | ε | ε | 1,5 | 0,8 | 3,7 |
| 44 | 4,3 | 24,3 | 47 | 71,7 | 17,4 | 1,3 | 0,3 | 18,9 | 4,6 | 4,9 | 1,2 | 3,1 | 0,7 | ε | ε | 0,9 |
| 45 | 4,1 | 21,9 | 49 | 77,8 | 17 | 2,3 | 0,5 | 13,7 | 3 | 4 | 0,9 | 2,1 | 0,5 | ε | ε | 0,7 |
| 46 | 4,3 | 27 | 63 | 85 | 23 | 2 | 0,5 | 9,6 | 2,6 | 2,2 | 0,6 | 1 | 0,3 | 0,2 | ε | 0,7 |

Tableau VII

| EX | BD mM | EtOH mM | $PdCl_2$ | HCl | BD/Pd molaire | HCl/Pd molaire | Additif | Additif/ Pd molaire | PCO bars |
|---|---|---|---|---|---|---|---|---|---|
| 47 | 213 | 391 | 0,423 | 21,1 | 504 | 50 | 0 | 0 | ↗ 80 |
| 1 | 213 | 391 | 0,423 | 21,1 | 504 | 50 | 0 | 0 | 155 |
| 48 | 259 | 391 | 0,423 | 21,1 | 614 | 50 | $NMe_4^+Cl^-$ | 2 | ↗ 80 |
| 49 | 250 | 391 | 0,423 | 21,1 | 591 | 50 | $NBu^+Cl^-$ | 2 | 100 |
| 50 | 230 | 391 | 0,423 | 21,1 | 544 | 50 | $NBu_4^+Cl^-$ | 2 | 120 |
| 12 | 278 | 391 | 0,423 | 21,1 | 657 | 50 | $NBu_4^+Cl^-$ | 2 | ↗158 |

Tableau VII'

| EX | PCO bar | TT | A | P3 | | P' | | C9 | | HC8 | | ROC4 | | C6 | | RRCl % |
|---|---|---|---|---|---|---|---|---|---|---|---|---|---|---|---|---|
| | | | | RT | RR | RT | RR | RT | RR | RT | RR | RT | RR | RT | RR | |
| 47 | 80 | 11,1 | 17 | 62,7 | 6,9 | 2 | 0,2 | 1,3 | 0,1 | 20,3 | 2,2 | 13,6 | 1,5 | ε | ε | 3,2 |
| 1 | 155 | 37,4 | 84 | 89,6 | 33,5 | 2,6 | 1 | 2,5 | 0,9 | 3,5 | 1,3 | 1,8 | 0,7 | ε | ε | 5,5 |
| 48 | ↗ 80 | 16,1 | 35 | 72,1 | 11,6 | 2,7 | 0,4 | 2,4 | 0,4 | 16,6 | 2,7 | 6,2 | 1 | ε | ε | 3 |
| 49 | 100 | 23,9 | 61 | 86,5 | 20,7 | 2,5 | 0,6 | 3,5 | 0,8 | 4,7 | 1,1 | 1,7 | 0,4 | 0,8 | 0,2 | 3,7 |
| 50 | 120 | 36,2 | 88 | 89,5 | 32,4 | 1,9 | 0,7 | 3,5 | 1,3 | 3,2 | 1,2 | 1,3 | 0,5 | 0,6 | 0,2 | 4 |
| 12 | ↗158 | 50,4 | 151 | 91,3 | 46 | 3,1 | 1,6 | 1,6 | 0,8 | 2,3 | 1,2 | 1,1 | 0,6 | 0,4 | 0,2 | 6,2 |

Tableau VIII

| EX | BD mM | EtOH mM | $PdCl_2$ mM | Cocatalyseur | BD/Pd molaire | HCl/Pd molaire | $NBu_4^+Cl^-$/ Pd molaire | T°C | Durée h | PCO bars |
|---|---|---|---|---|---|---|---|---|---|---|
| 51 | 194 | 391 | 0,423 | HCl | 460 | 50 | 0 | 100 | 2 | ↗142 |
| 52 | 250 | 391 | 0,33 | 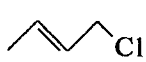 | 758 | 50 | 3 | 100 | 2 | 140 |
| 1 | 213 | 391 | 0,423 | HCl | 504 | 50 | 0 | 120 | 2 | ↗155 |
| 25 | 259 | 391 | 0,423 | HCl | 613 | 50 | 3 | 120 | 2 | 145 |

Tableau VIII'

| EX | T °C | TT % | A | P 3 RT % | P 3 RR % | P' RT % | P' RR % | C 9 RT % | C 9 RR % | HC 8 RT % | HC 8 RR % | ROC 4 RT % | ROC 4 RR % | C 6 RT % | C 6 RR % | RRCl % |
|---|---|---|---|---|---|---|---|---|---|---|---|---|---|---|---|---|
| 51 | 100 | 21,3 | 43 | 90,6 | 19,3 | 2,4 | 0,5 | 1,2 | 0,3 | 2,1 | 0,5 | 2,9 | 0,6 | 0,6 | 0,12 | 2,6 |
| 52 | 100 | 20,6 | 70 | 90,1 | 18,6 | 2,5 | 0,5 | 2,3 | 0,5 | 2,9 | 0,6 | 1,6 | 0,3 | ε | ε | 0,9 |
| 1 | 120 | 37,4 | 84 | 89,6 | 33,5 | 2,6 | 1 | 2,5 | 0,9 | 3,5 | 1,3 | 1,8 | 0,7 | ε | ε | 5,5 |
| 25 | 120 | 55,3 | 156 | 91,8 | 50,9 | 2,5 | 1,4 | 1,8 | 1 | 1,3 | 0,7 | ε | ε | 1,5 | 0,8 | 3,7 |

Tableau IX

| EX | BD mM | $CH_3OH$ mM | $PdCL_2$ mM | 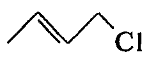 mM | BD/Pd molaire | HCl/Pd molaire | $NMeOct_3^+Cl^-$/Pd molaire | T°C | Durée h | PCO bars |
|---|---|---|---|---|---|---|---|---|---|---|
| 53 | 1340 | 3290 | 2,7 | 320 | 500 | 118 | 0 | 120 | 22 | 145 |
| 54 | 278 | 391 | 0,423 | 21,1 | 657 | 50 | 2 | 120 | 2 | 145 |

Tableau IX'

| EX | TT % | A | P 3 RT % | P 3 RR % | P' RT % | P' RR % | C 9 RT % | C 9 RR % | HC 8 RT % | HC 8 RR % | ROC 4 RT % | ROC 4 RR % | C 6 RT % | C 6 RR % | RRCl % |
|---|---|---|---|---|---|---|---|---|---|---|---|---|---|---|---|
| 53 | 6 | 1,5 | Faible | | | | | | | | | | | | |
| 54 | 16,8 | 34 | 61,3 | 10,3 | 1,7 | 0,3 | 17,7 | 3 | 13 | 2,2 | 6,2 | 1 | | | |

Tableau X

| EX | diène | mM diène | EtOH mM | $PdCl_2$ mM | HCl mM | BD/Pd molaire | HCl/Pd molaire | $NBu_4^+Cl^-$/Pd molaire | t°C | durée h | PCO bars |
|---|---|---|---|---|---|---|---|---|---|---|---|
| 55 | 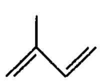 | 278 | 391 | 0,423 | 21,1 | 658 | 50 | 2 | 120 | 2 | 145 |
| 56 | 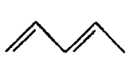 | 93 | 130 | 0,141 | 7 | 658 | 50 | 2 | 120 | 2 | 145 |

Tableau X'

| EX | TT % | A | 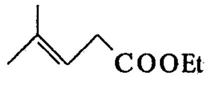 | | 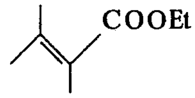 | | Ethoxy pentènes | | Ethoxy décènes | | Lactones | | RR Cl % |
|---|---|---|---|---|---|---|---|---|---|---|---|---|---|
| | | | RT % | RR % | RT % | RR % | RT % | RR % | RT % | RR % | RT % | RR % | |
| 55 | 78,5 | 218 | 84,5 | 66,3 | 4,9 | 3,9 | 4 | 3,1 | 5,9 | 4,8 | 1,8 | 1,4 | 1,1 |

Tableau X' (Suite)

| EX | TT % | A | 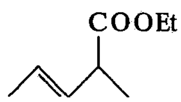 | | Ethoxy pentènes | | Ethoxy décènes | | Dimères | | RR Cl % |
|---|---|---|---|---|---|---|---|---|---|---|---|
| | | | RT % | RR % | RT % | RR % | RT % | RR % | RT % | RR % | |
| 56 | 37 | 26 | 20,8 | 7,7 | 65 | 24 | 0,9 | 0,3 | 1,4 | 0,5 | 3,1 |

## Revendications

1. Procédé de préparation d'esters d'acides carboxyliques $\beta,\gamma$-insaturés par carbonylation d'un diène conjugué par de l'oxyde de carbone, en présence de l'alcool correspondant à l'ester recherché, d'un hydracide halogéné et d'un catalyseur au palladium, à une température comprise entre 50 et 150°C et sous une pression d'oxyde de carbone de 50 à 300 bars, ledit procédé étant caractérisé en ce que:

- l'opération de carbonylation est réalisée en outre en présence d'un sel d'onium quaternaire d'un élément du groupe V B choisi parmi l'azote, le phosphore et l'arsenic, ledit élément étant quadricoordiné à des atomes de carbone et ledit sel présentant un anion choisi parmi les bases »dures« ou »intermédiaires«;
- le catalyseur au palladium est constitué par:
  - du palladium métal,
  - un oxyde de palladium,
  - ou un sel ou complexe de palladium dont l'anion coordiné au cation palladium est une base »dure« ou »intermédiaire«;
- le rapport molaire hychaude halogéné/palladium est d'au moins 5;
- le rapport molaire cationonium/palladium est d'au moins 0,5.

2. Procédé selon la revendication 1, caractérisé en ce que le sel d'onium quaterniaire présente un cation onium quaternaire répondant à l'une des formules I à III suivantes:

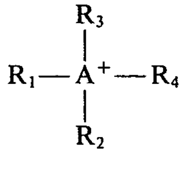 (I)

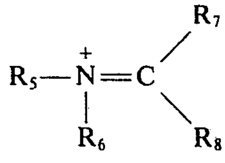 (II)

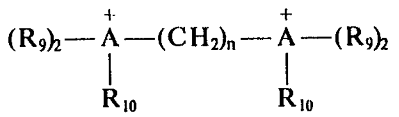

(III)

où:

- A représente de l'azote, du phosphore ou de l'arsenic
- $R_1$, $R_2$, $R_3$, $R_4$ sont identiques ou différents et représentent:
  - un radical alkyle linéaire ou ramifié contenant de 1 à 16 atomes de carbone, éventuellement substitué par un groupe phényle, hydroxy, halogéno, nitro, alcoxy ou alcoxycarbonyle;
  - un radical alcényle linéaire ou ramifié contenant de 2 à 12 atomes de carbone;
  - un radical aryle contenant de 6 à 10 atomes de carbone, éventuellement substitué par un ou des radicaux alkyles contenant de 1 à 4 atomes de carbone, alcoxy, alcoxycarbonyle ou halogéno;
  - deux desdits radicaux $R_1$ à $R_4$ pouvant former ensemble un radical alkylène, alcénylène ou alcadiènylène, linéaire ou ramifié et contenant de 3 à 6 atomes de carbone;
- $R_5$, $R_6$, $R_7$, $R_8$ sont identiques ou différents et représentent:
  - un radical alkyle linéaire ou ramifié contenant de 1 à 4 atomes de carbone;
  - les radicaux $R_7$ et $R_8$ pouvant former ensemble un radical alkylène contenant de 3 à 6 atomes de carbone;
  - les radicaux $R_6$ et $R_7$ ou $R_6$ et $R_8$ pouvant former ensemble un radical alkylène, alcénylène ou alcadiénylène, contenant 4 atomes de carbone et constituant avec N un hétérocycle azoté;
- $R_9$ représente un radical alkyle linéaire ou ramifié contenant de 1 à 4 atomes de carbone ou un radical phényle;
- $R_{10}$ représente:
  - un radical alkyle linéaire ou ramifié contenant de 1 à 4 atomes de carbone, semblable ou différent de $R_9$;
  - un radical alcényle linéaire ou ramifié, contenant de 2 à 12 atomes de carbone,
- n représente un nombre entier supérieur ou égal à 1 et inférieur ou égal à 10.

3. Procédé selon la revendication 2, caractérisé en ce que lorsqu'ils représentent des radicaux alcényles, les groupes $R_2$, et $R_{10}$ contiennent de 4 à 8 atomes de carbone.

4. Procédé selon la revendication 3, caractérisé en ce que les radicaux $R_2$ et $R_{10}$ sont des radicaux alcényles dérivés du diène conjugué mis en oeuvre.

5. Procédé selon la revendication 2, caractérisé en ce que n est un nombre entier inférieur ou égal à 6.

6. Procédé selon la revendication 1, caractérisé en ce que l'anion dudit sel d'onium est un anion

$PO_4^{3-}$, $HPO_4^{2-}$, $H_2PO_4^-$, $CH_3SO_3^-$, 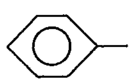 $-SO_3^-$, $NO_3^-$, $SO_4^{2-}$, $PF_6^-$, $Cl^-$, ou $Br^-$

7. Procédé selon l'une quelconque des revendication 2 à 5 caractérisé en ce que ledit cation est un cation:

tétraméthylammonium,
tétraéthylammonium,
tétradodécylammonium,
tétrabutylammonium,
méthyltrioctylammonium,
hexadécyltriméthylammonium,
méthyltriphénylammonium,
benzyltriméthylammonium,
butène-2 yltriéthylammonium,
tétraméthylphosphonium,
tétrabutylphosphonium,
hexadécyltributylphosphonium,
éthyltriméthylphosphonium,
méthyltriphénylphosphonium,
hexadécylpyridinium,

bis(butène-2 yldiméthylammonium)-1,3 propane,
ou tétraphénylarsonium.

8. Procédé selon la revendication 1, caractérisé en ce que le catalyseur au palladium est un sel de palladium ou un complexe $\pi$-allylique de palladium dont l'anion coordiné au cation palladium est un anion carboxylate, $SO_4^{2-}$, $NO_3^-$, acétylacétonate, chlorure ou bromure ou un complexe de palladium zéro comprenant des ligands organiques ne contenant pas d'éléments du groupe VB.

9. Procédé selon la revendication 1, caractérisé en ce que l'opération de carbonylation est réalisée en présence de quantités de réactifs correspondant aux rapports molaires suivants:

alcool/diène conjugué: de 0,5 à 10,
diène conjugué/palladium: de 100 à 2500.

10. Procédé selon la revendication 9 caractérisé en ce que lesdits rapports molaires sont les suivants:

alcool/diène conjugué: de 0,8 à 5,
diène conjugué/palladium: de 250 à 1200,
hydracide halogéné/Palladium: 10 à 150,
cation onium/Palladium: de 1 à 15.

11. Procédé selon la revendication 10, caractérisé en ce que le rapport molaire hydracide halogéné/palladium est compris entre 20 et 100.

12. Procédé de préparation de pentène-3 oate d'éthyle par carbonylation du butadiène par de l'oxyde de carbone en présence d'éthanol, d'acide chlorhydrique et d'un catalyseur au palladium, à une température comprise entre 50 et 150°C et sous une pression d'oxyde de carbone de 50 à 300 bars, ledit procédé étant caractérisé en ce que:

— le mileu de carbonylation contient en outre un sel d'onium quaternaire d'un élément du groupe VB choisi parmi l'azote, le phosphore et l'arsenic, ledit élément étant quadricoordiné à des atomes de carbone et ledit sel présentant un anion choisi parmi les bases »dures« ou »intermédiaires«
— le catalyseur au palladium est du chlorure de palladium II ou du bis [$\pi$-allyl palladium (II) chlorure]
— les différents réactifs sont mis en oeuvre selon les rapports molaires suivants:
éthanol/butadiène: de 1 à 5,
butadiène/palladium: de 250 à 1200,
HCl/Palladium: de 10 à 150,
cation onium/palladium: de 1 à 15.

13. Procédé selon la revendication 12 caractérisé en ce que le rapport molaire butadiène/palladium est compris entre 500 et 700 et le rapport molaire hydracide halogéné/palladium est compris entre 20 et 100.

**Patentansprüche**

1. Verfahren zur Herstellung von Estern von $\beta$, $\gamma$-ungesättigten Carbonsäuren durch Carbonylieren eines konjugierten Diens mit Kohlenoxid in Gegenwart des dem angestrebten Ester entsprechenden Alkohols, einer Halogenwasserstoffsäure und eines Palladiumkatalysators bei einer Temperatur von 50 bis 150°C und unter einem Kohlenoxiddruck von 50 bis 300 bar, dadurch gekennzeichnet, daß

— die Carbonylierung zusätzlich in Gegenwart eines quaternären Oniumsalzes eines Elementes der Gruppe V B (des Periodensystems) ausgewählt unter Stickstoff, Phosphor und Arsen durchgeführt wird, wobei das Element vierfach an Kohlenstoffatome koordiniert ist und das Salz ein unter den »harten« oder »mittleren« Basen ausgewähltes Anion aufweist,
— der Palladiumkatalysator besteht aus:

metallischem Palladium,
einem Palladiumoxid,
oder einem Palladiumsalz oder -komplex, dessen dem Palladiumkation koordiniertes Anion eine »harte« oder »mittlere« Base ist,

— und das Molverhältnis von Halogenwasserstoffsäure zu Palladium mindestens 5 und
— das Molverhältnis von Oniumkation zu Palladium mindestens 0,5 beträgt.

2. Verfahren nach Anspruch 1, dadurch gekennzeichnet, daß das quaternäre Oniumsalz ein quaternäres Oniumkation aufweist, das einer der folgenden Formeln I bis III entspricht:

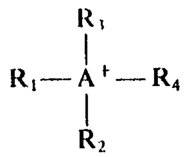

(I)

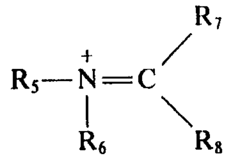

(II)

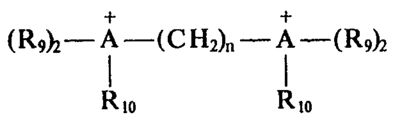

(III)

in denen

- A Stickstoff, Phosphor oder Arsen bedeutet,
- $R_1$, $R_2$, $R_3$ und $R_4$ gleich oder verschieden sind und (jeweils)
  - einen linearen oder verzweigten Alkylrest mit 1 bis 16 Kohlenstoffatomen, der gegebenenfalls durch ein Halogenatom, eine Phenyl-, Hydroxy-, Nitro-, Alkoxy- oder Alkoxycarbonylgruppe substituiert ist,
  - einen linearen oder verzweigten Alkenylrest mit 2 bis 12 Kohlenstoffatomen oder
  - einen Arylrest mit 6 bis 10 Kohlenstoffatomen, der gegebenenfalls durch mindestens eine Alkylgruppe mit 1 bis 4 Kohlenstoffatomen, eine Alkoxygruppe oder Alkoxycarbonylgruppe oder ein Halogenatom substituiert ist, bedeuten und
  - zwei der Reste $R_1$ bis $R_4$ zusammen einen linearen oder verzweigten Alkylen-, Alkenyl- oder Alkadienylenrest mit 3 bis 6 Kohlenstoffatomen bilden können,
- $R_5$, $R_6$, $R_7$ und $R_8$ gleich oder verschieden sind und (jeweils)
  - einen linearen oder verzweigten Alkylrest mit 1 bis 4 Kohlenstoffatomen bedeuten,
  - die Reste $R_7$ und $R_8$ zusammen einen Alkylenrest mit 3 bis 6 Kohlenstoffatomen bilden können und
  - die Reste $R_6$ und $R_7$ oder $R_6$ und $R_8$ zusammen einen Alkylen-, Alkenylen- oder Alkadienylenrest mit 4 Kohlenstoffatomen bilden können, der zusammen mit N einen Stickstoff-Heterocyclus ergibt,
- $R_9$ einen linearen oder verzweigten Alkylrest mit 1 bis 4 Kohlenstoffatomen oder einen Phenylrest bedeutet,
- $R_{10}$
  - einen linearen oder verzweigten Alkylrest mit 1 bis 4 Kohlenstoffatomen, gleich oder verschieden von $R_9$, oder
  - einen linearen oder verzweigten Alkenylrest mit 2 bis 12 Kohlenstoffatomen, bedeutet und
- n eine ganze Zahl größer oder gleich 1 und kleiner oder gleich 10 ist.

3. Verfahren nach Anspruch 2, dadurch gekennzeichnet, daß die Reste $R_2$ und $R_{10}$, wenn sie Alkenylgruppen bedeuten, 4 bis 8 Kohlenstoffatome aufweisen.
4. Verfahren nach Anspruch 3, dadurch gekennzeichnet, daß die Reste $R_2$ und $R_{10}$ von dem eingesetzten konjugierten Dien abgeleitete Alkenylreste sind.
5. Verfahren nach Anspruch 2, dadurch gekennzeichnet, daß n eine ganze Zahl kleiner gleich 6 ist.
6. Verfahren nach Anspruch 1, dadurch gekennzeichnet, daß das Anion des Oniumsalzes eines der folgenden Anionen ist:

$PO_4^{3-}$, $HPO_4^{2-}$, $H_2PO_4^-$, $CH_3SO_3^-$, 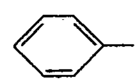—$SO_3^-$, $NO_3^-$, $SO_4^{2-}$, $PF_6^-$, $Cl^-$, oder $Br^-$.

7. Verfahren nach einem der Ansprüche 2 bis 5, dadurch gekennzeichnet, daß das Kation eines der folgenden Kationen ist:

Tetramethylammonium,
Tetraethylammonium,
Tetradodecylammonium,
Tetrabutylammonium,
Methyltrioctylammonium,
Hexadecyltrimethylammonium,
Methyltriphenylammonium,
Benzyltrimethylammonium,
2-Butenyltriethylammonium,
Tetramethylphosphonium,
Tetrabutylphosphonium,
Hexadecyltributylphosphonium,
Ethyltrimethylphosphonium,
Methyltriphenylphosphonium,
Hexadecylpyridinum,
1,3-Bis-(2-butenyldimethylammonium)-propan oder
Tetraphenylarsonium.

8. Verfahren nach Anspruch 1, dadurch gekennzeichnet, daß der Palladiumkatalysator ein Palladiumsalz oder ein $\pi$-Allylkomplex von Palladium ist, dessen dem Palladiumkation koordiniertes Anion ein Carboxylat-, $SO_4^{2-}$, $NO_3^-$, Acetylacetonat-, Chlorid- oder Bromidanion ist, oder ein Komplex von 0wertigem Palladium, umfassend organische Liganden, die keine Elemente der Gruppe V B (des Periodensystems) enthalten.

9. Verfahren nach Anspruch 1, dadurch gekennzeichnet, daß die Carbonylierungsreaktion in Anwesenheit von Mengen der Reaktionspartner durchgeführt wird, die folgenden Molverhältnissen entsprechen:

Alkohol/konjugiertes Dien: 0,5 bis 10,
konjugiertes Dien/Palladium: 100 bis 2500.

10. Verfahren nach Anspruch 9, dadurch gekennzeichnet, daß folgende Molverhältnisse eingehalten werden:

Alkohol/konjugiertes Dien: 0,8 bis 5,
konjugiertes Dien/Palladium: 250 bis 2200,
Halogenwasserstoffsäure/Palladium: 10 bis 150,
Oniumkation/Palladium: 1 bis 15.

11. Verfahren nach Anspruch 10, dadurch gekennzeichnet, daß das Molverhältnis von Halogenwasserstoffsäure/Palladium 20 bis 100 beträgt.

12. Verfahren zur Herstellung von Ethyl-3-pentenoat durch Carbonylieren von Butadien mit Kohlenoxid in Gegenwart von Ethanol, Chlorwasserstoff und einem Palladiumkatalysator bei einer Temperatur von 50 bis 150°C und unter einem Kohlenoxiddruck von 50 bis 300 bar, dadurch gekennzeichnet, daß:

- das Carbonylierungsmedium zusätzlich ein quaternäres Oniumsalz eines Elementes der Gruppe V B (des Periodensystems), ausgewählt aus Stickstoff, Phosphor und Arsen, enthält, wobei dieses Element 4fach an Kohlenstoffatome koordiniert ist und das Salz ein unter den »harten« oder »mittleren« Basen ausgewähltes Anion aufweist,
- der Palladiumkatalysator Palladium (II)chlorid oder [Bis-allyl-palladium(II)chlorid] ist und
- die verschiedenen Reaktionspartner entsprechend folgenden Molverhältnissen eingesetzt werden:

Ethanol/Butadien: 1 bis 5,
Butadien/Palladium: 250 bis 1200,
HCl/Palladium: 10 bis 150,
Oniumkation/Palladium: 1 bis 15.

13. Verfahren nach Anspruch 12, dadurch gekennzeichnet, daß das Molverhältnis von Butadien/Palladium 500 bis 700 und das Molverhältnis von Halogenwasserstoffsäure/Palladium 20 bis 100 beträgt.

**Claims**

1. Process for the preparation of esters of $\beta,\gamma$-unsaturated carboxylic acids, by carbonylation of a conjugated diene with carbon monoxide, in the presence of the alcohol corresponding to the desired ester, of a hydrogen halide and of a palladium catalyst, at a temperature of between 50 and 150°C under a carbon monoxide pressure of 50 to 300 bars, the said process being characterised in that:
the carbonylation operation is moreover carried out in the presence of a quaternary onium salt of an element of group V B, chosen from among nitrogen, phosphorus and arsenic, the said element being quadricoordinated in respect of the carbon atoms and the said salt having an anion chosen from among the »hard« or »intermediate« bases,
the palladium catalyst consists of palladium metal, a palladium oxide or a palladium salt or complex in which the anion coordinated with the palladium cation is a »hard« or »intermediate« base,
the molar ratio of hydrogen halide to palladium is at least 5 and
the molar ratio of onium cation to palladium is at least 0.5.

2. Process according to Claim 1, characterised in that the quaternary onium salt has a quaternary onium cation corresponding to one of the following formulae I to III:

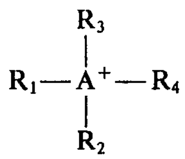

(I)

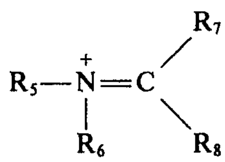

(II)

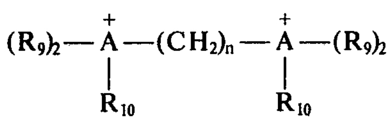

(III)

where

A represents nitrogen, phosphorus or arsenic
$R_1$, $R_2$, $R_3$ and $R_4$ are identical or different and represent a linear or branched alkyl radical containing from 1 to 16 carbon atoms and optionally substituted by a phenyl, hydroxyl, halogen, nitro, alkoxy or alkoxycarbonyl group, a linear or branched alkenyl radical containing from 2 to 12 carbon atoms, or an aryl radical containing from 6 to 10 carbon atoms and optionally substituted by one or more alkyl radicals containing form 1 to 4 carbon atoms, alkoxy, alkoxycarbonyl or halogen, and two of the said radicals $R_1$ to $R_4$ can together form a linear or branched alkylene, alkenylene or alkadienylene radical containing 3 to 6 carbon atoms,
$R_5$, $R_6$, $R_7$ and $R_8$ are identical or different and represent a linear or branched alkyl radical containing from 1 to 4 carbon atoms, the radicals $R_7$ and $R_8$ can together form an alkylene radical containing from 3 to 6 carbon atoms and the radicals $R_6$ and $R_7$ or $R_6$ and $R_8$ can together form an alkylene, alkenylene or alkadienylene radical containing 4 carbon atoms and forming, together with N, a nitrogen-containing heterocyclic ring,
$R_9$ represents a linear or branched alkyl radical containing from 1 to 4 carbon atoms, or a phenyl radical,
$R_{10}$ represents a linear or branched alkyl radical containing from 1 to 4 carbon atoms which may be similar to or different from $R_9$ or a linear or branched alkenyl radical containing from 2 to 12 carbon atoms, and
n represents an integer greater than or equal to 1 and less than or equal to 10.

3. Process according to Claim 2, characterised in that if they represent alkenyl radicals, the groups $R_2$ and $R_{10}$ contain from 4 to 8 carbon atoms.

4. Process according to Claim 3, characterised in that the radicals $R_2$ and $R_{10}$ are alkenyl radicals derived from the conjugated diene employed.

5. Process according to Claim 2, characterised in that n is an integer less than or equal to 6.

6. Process according to Claim 1, characterised in that the anion of the said onium salt is a

$PO_4^{3-}$, $HPO_4^{2-}$, $H_2PO_4^-$, $CH_3SO_3^-$, 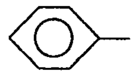$-SO_3^-$, $NO_3^-$, $SO_4^{2-}$, $PF_6^-$, $Cl^-$, or $Br^-$ anion.

7. Process according to any one of Claims 2 to 5, characterised in that the said cation is a

tetramethylammonium,
tetraethylammonium,
tetradodecylammonium,
tetrabutylammonium,
methyltrioctylammonium,
hexadecyltrimethylammonium,
methyltriphenylammonium,
benzyltrimethylammonium,
but-2-enyltriethylammonium,
tetramethylphosphonium,
tetrabutylphosphonium,
hexadecyltributylphosphonium,
ethyltrimethylphosphonium,
methyltriphenylphosphonium,
hexadecylpyridinium,
1,3-bis-(but-2-enyldimethylammonium)-propane or
tetraphenylarsonium cation.

8. Process according to Claim 1, characterised in that the palladium catalyst is a palladium salt or a $\pi$-allyl complex of palladium wherein the anion coordinated to the palladium cation is a carboxylate, $SO_4^{2-}$, $NO_3^-$, acetylacetonate, chloride or bromide anion or a complex of zero-valency palladium comprising organic ligands which do not contain elements of group VB.

9. Process according to Claim 1, characterised in that the carbonylation operation is carried out in the presence of amounts of reactants corresponding to the following molar ratios:

alcohol/conjugated diene: from 0.5 to 10,
conjugated diene/palladium: from 100 to 2500.

10. Process according to Claim 9, characterised in that the said molar ratios are as follows:

alcohol/conjugated diene: from 0.8 to 5,
conjugated diene/palladium: from 250 to 1200,
hydrogen halide/palladium: 10 to 150,
onium cation/palladium: from 1 to 15.

11. Process according to Claim 10, characterised in that the molar ratio of hydrogen halide/palladium is between 20 and 100.

12. Process for the preparation of ethyl pent-3-enoate by carbonylation of butadiene with carbon monoxide in the presence of ethanol, hydrochloric acid and a palladium catalyst, at a temperature of between 50 and 150°C and under a carbon monoxide pressure of 50 to 300 bars, the said process being characterised in that:

the carbonylation medium moreover contains a quaternary onium salt of an element of group VB chosen from among nitrogen, phosphorus and arsenic, the said element being quadri-coordinated with respect to carbon atoms and the said salt having an anion chosen from among the »hard« or »intermediate« bases,
the palladium catalyst is palladium-II chloride or bis-[$\pi$-allyl-palladium-(II) chloride] and
the various reactants are employed in the following molar ratios:

ethanol/butadiene: from 1 to 5,
butadiene/palladium: from 250 to 1200,
HCl/palladium: from 10 to 150,
onium cation/palladium: from 1 to 15.

13. Process according to Claim 12, characterised in that the molar ratio of butadiene/palladium is between 500 and 700 and the molar ratio of hydrogen halide/palladium is between 20 and 100.